(19) Europäisches Patentamt
European Patent Office
Office européen des brevets

(11) EP 1 524 986 B1

(12) **EUROPÄISCHE PATENTSCHRIFT**

(45) Veröffentlichungstag und Bekanntmachung des Hinweises auf die Patenterteilung:
**13.07.2011 Patentblatt 2011/28**

(21) Anmeldenummer: **03784123.6**

(22) Anmeldetag: **31.07.2003**

(51) Int Cl.:
***A61K 33/24*** (2006.01) ***A61P 35/00*** (2006.01)

(86) Internationale Anmeldenummer:
**PCT/EP2003/008475**

(87) Internationale Veröffentlichungsnummer:
**WO 2004/014401 (19.02.2004 Gazette 2004/08)**

(54) **METALLCLUSTER-NANOVERBINDUNGEN ZUR BEHANDLUNG VON TUMORERKRANKUNGEN**

METAL CLUSTER NANO-COMPOUNDS FOR TREATING TUMOR DISEASES

NANOCOMPOSES DE CLUSTERS METALLIQUES DESTINES AU TRAITEMENT DE MALADIES TUMORALES

(84) Benannte Vertragsstaaten:
**AT BE BG CH CY CZ DE DK EE ES FI FR GB GR HU IE IT LI LU MC NL PT RO SE SI SK TR**

(30) Priorität: **02.08.2002 DE 10235602**

(43) Veröffentlichungstag der Anmeldung:
**27.04.2005 Patentblatt 2005/17**

(73) Patentinhaber: **Universität Duisburg-Essen**
**45141 Essen (DE)**

(72) Erfinder:
- **SCHMID, Günter**
  **42555 Velbert (DE)**
- **KUHN, Hubert**
  **42697 Solingen (DE)**
- **TSOLI, Maria**
  **Kogarah, Sydney, NSW 2217 (AU)**

(74) Vertreter: **Gesthuysen, von Rohr & Eggert**
**Patentanwälte**
**Huyssenallee 100**
**45128 Essen (DE)**

(56) Entgegenhaltungen:

| | |
|---|---|
| **EP-A- 0 195 147** | **WO-A-01/77121** |
| **WO-A-94/21288** | **US-A- 5 360 895** |
| **US-A- 5 521 289** | **US-A- 6 037 366** |
| **US-B1- 6 369 206** | |

- **LIU, Y. ET AL.: "Gold-Cluster Degradation by the Transition of B-DNA into A-DNA and the Formation of Nanowires" ANGEWANDTE CHEMIE, INTERNATIONAL EDITION, Bd. 42, Nr. 25, 30. Juni 2003 (2003-06-30), Seiten 2853-2857, XP002262744**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 524 986 B1

Printed by Jouve, 75001 PARIS (FR)

**Beschreibung**

Die vorliegende Erfindung betrifft Metalleluster-Nanoverbindungen einschließlich ihrer physiologisch verträglichen Salze zur prophylaktischen und/oder therapeutischen (kurativen) Behandlung von Erkrankungen des menschlichen oder tierischen Körpers, insbesondere von benignen wie malignen Tumor- bzw. Krebserkrankungen.

Insbesondere betrifft die vorliegende Erfindung die Verwendung von Metallcluster-Nanoverbindungen einschließlich ihrer physiologisch verträglichen Salze als pharmazeutische Wirkstoffs bzw. Arzneimittelwirkstoffe, insbesondere zur Herstellung von Arzneimitteln zur prophylaktischen und/oder therapeutischen (kurativen) Behandlung von Tumor- bzw. Krebserkrankungen. Gleichermaßen betrifft die vorliegende Erfindung Arzneimittel bzw, pharmazeutische Zusammensetzungen, welche diese Metallcluster-Nanoverbindungen einschließlich ihrer physiologisch verträglichen Salze enthalten.

Tumor- und Krebserkrankungen sind kein einheitliches Krankheitsbild, sondern Oberbegriffe für eine Vielzahl verschiedener Formen gutartiger, benigner wie bösartiger, maligner Erkrankungen, Nahezu jedes Gewebe unseres Körpers kann krebsige Entartungen hervorbringen, manchmal sogar mehrere unterschiedliche Typen. Jedes der Leiden wiederum hat seine eigenen Merkmale. Die Ursachen, die zu diesen Erkrankungen führen, sind oftmals sehr heterogen,

Trotz dieser Verschiedenartigkeit entstehen nahezu alte Tumore bzw. krebsigen Entartungen durch sehr ähnliche, grundlegende molekulare bzw. zelluläre Prozesse. In den letzten zwei Jahrzehnten hat die Forschung erstaunliche Erkenntnisfortschritte erbracht, was die fundamentalsten Prozesse des Krebs- bzw. Tumorgeschehens auf molekularer Ebene anbelangt.

Träger der Erbinformationen sind die DNA-Moleküle der Chromosomen im Zellkern. Zwei Klassen von Genen, die zusammen nur einen Meinen Anteil der gesamten Ausstattung einer Zelle ausmachen, spielen für die Krebsentstehung eine wesentliche Rolle, nämlich insbesondere Proto-Onkogene (Krebsgen-Vorläufer) und Tumorsuppressor-Gene (tumorunterdrückende Gene). In ihrer normalen Form dirigieren sie den Lebenszyklus der Zelle, sie steuern die verwikkelte Abfolge von Vorgängen, durch die sich eine Zelle vergrößert und bei Bedarf teilt. Während Proto-Onkogene das Zellwachstum fördern, wird es durch Tumorsuppressor-Gene gebremst. Zusammen sind diese beiden Klassen von Genen für einen Großteil der unkontrollierten Zellvermehrungsprozesse in menschlichen Tumoren verantwortlich: Wenn z. B. ein Proto-Onkogen in der Regulator- oder in der Strukturregion mutiert, kann es passieren, daß nun zuviel von seinem wachstumsfördernden Protein hergestellt wird oder daß dieses nun übermäßig aktiv ist; aus dem Proto-Onkogen ist dann ein krebsbegünstigendes Onkogen geworden, das die Zellen zu übermäßiger Vermehrung anregt. Demgegenüber tragen Tumorsuppressor-Gene zur Krebsentstehung bei, wenn sie durch Mutationen inaktiviert werden; als Folge verliert die Zelle funktionsfähige Suppressor-Proteine und damit entscheidende Wachstumsbremsen, die sie normalerweise an einer unangemessenen Vermehrung hindern.

In normalen Körperzellen ist ein Notmechanismus gegen unbegrenzte Vermehrung eingebaut, es handelt sich dabei um eine Art Zählwerk, das jede Zellteilung registriert und nach einer bestimmten Anzahl von Generationen Einhalt gebietet. Nach einer bestimmten, in etwa voraussagbaren Zahl von Zellteilungen bzw. Verdoppelungen hört das Wachstum normaler Zellen auf. Dieser Prozeß wird als Zellalterung oder Seneszenz bezeichnet.

Für diesen Prozeß der Zellalterung oder Senenszenz auf molekularer Ebene verantwortlich sind die DNA-Segmente an den Enden der Chromosomen, die sogenannten Telomere. Sie registrieren sozusagen, wieviele Vermehrungszyklen eine Zellpopulation durchläuft und leiten ab einem bestimmten Zeitpunkt die Seneszenz bzw. die Krise ein. Dadurch begrenzen Sie die Fähigkeit einer Zellpopulation, uneingeschränkt zu wachsen.

Der zuvor beschriebene Schutzmechanismus ist im Zuge der Entartung bei den meisten Krebs- bzw. Tumorzellen außer Kraft gesetzt. Daher besteht das Ziel vieler therapeutischer Ansätze darin, das Zellwachstum oder die Zellteilung von Tumor- bzw. Krebszellen zu hemmen oder zu beenden, insbesondere möglichst eine Blockierung oder sogar Zerstörung der DNA von Tumor- bzw. Krebszellen zu induzieren. Zu diesem Zweck kommen beispielsweise Metallverbindungen des Platins oder Rutheniums, wie z. B. das cis-Diaminodichloro-Platin(II) ("cis-Platin"), zum Einsatz.

Wechselwirkungen zwischen Metallen und biologischen Makromoleküle einschließlich Proteinen, Polysacchariden und Nukleinsäuren sind von besonderem Interesse, da sie für eine Vielzahl natürlicher und technischer Abläufe von entscheidender Bedeutung sind. Diese reichen von Wechselwirkungen zwischen hochspezifischen Metallcofaktoren mit bestimmten Proteinen bis hin zur Biosorption von Schwermetallen durch Polysaccharidhydrogele.

Die einmaligen Eigenschaften der DNA haben zu einer Entwicklung von neuen Materialien, insbesondere im Bereich der Medizin, geführt. Die herkömmliche Antitumorforschung ist aber im wesentlichen auf die Wechselwirkungen zwischen Platin und Ruthenium enthaltenden Verbindungen mit den großen Furchen (*major grooves*) und kleinen Furchen (*minor grooves*) von Polynukleotiden gerichtet.

Die bislang eingesetzten Verbindungen haben aber zum Teil schwere Nebenwirkungen. So ist beispielsweise in bezug auf das cis-Platin, das an das Guanin der DNA und RNA bindet, seine extreme Nephrotoxizität bekannt, die schlimmstenfalls sogar zu Nekrosen führen kann. Des weiteren gibt es eine Reihe von cis-Platin-resistenten Tumoren, welche einer Therapie mit cis-Platin nicht zugänglich sind.

Die US-Patentschrift US 6,369,206 B1 betrifft beispielsweise Metall-Lipid-Moleküle der Formel M-Or-L, in denen

M ein Cluster oder Kolloid von Metallatomen aus der Gruppe von Gold, Silber, Platin, Palladium und deren Kombinationen darstellt, Or eine kovalent an die Metallatome gebundene organische Gruppe bezeichnet und L eine Lipideinheit, vorzugsweise Dipalmitoylphosphatidylethanolamin, darstellt, wobei M bevorzugt ein Cluster von etwa 50 bis 70 Goldatomen mit einem Durchmesser von etwa 1,4 nm darstellt.

Weiterhin werden in der US-Patentschrift US 5,521,289 B1 kleine organometallische Partikel aus einem Kern von 50 bis 70 Metallatomen beschrieben, an den organische Einheiten gebunden sind, wobei als Metallatome Gold, Silber, Platin, Palladium oder deren Kombinationen genannt werden, wobei die dort beschriebenen Verbindungen eine fluoreszierende Einheit enthalten.

Die US-Patentschrift US 5,360,819 A beschreibt sehr spezifische Antikörper- bzw. Antikörperfragment-Goldcluster, wobei die Goldcluster 6, 8, 9, 11, 13, 55 oder 67 Goldatome in ihrem inneren Kern enthalten und der Antikörper bzw. das Antikörperfragment über eine kovalente Bindung an die Goldcluster gekoppelt wird, wobei die Antikörper- bzw. Antikörperfragment-Goldcluster als Marker-Substanz in Analyseverfahren eingesetzt werden können.

Die Aufgabe der vorliegenden Erfindung besteht somit in der Auffindung bzw. Bereitstellung von Wirkstoffen und Arzneimitteln, die sich insbesondere für die Behandlung von Tumor- bzw. Krebserkrankungen, gegebenenfalls aber auch von anderen Erkrankungen des menschlichen oder tierischen Körpers, eignen.

Es wurde nun überraschend gefunden, daß Metallcluster-Nanoverbindungen von Übergangsmetallen und ihre physiologisch verträglichen Salze, Derivate, Isomere, Hydrate, Metabolite sowie Prodrugs sich zur prophylaktischen und/oder therapeutischen (kurativen) Behandlung von Erkrankungen des menschlichen oder tierischen Körpers, insbesondere von Tumor- bzw. Krebserkrankungen, eignen. Denn diese Verbindungen können unter bestimmten Voraussetzungen mit der DNA, insbesondere B-DNA, menschlicher oder tierischer Zellen, insbesondere von Tumor- oder Krebszellen, unter physiologischen Bedingungen in Wechselwirkung treten.

Zur Lösung der zuvor geschilderten Aufgabe schlägt die vorliegende Erfindung Metallcluster-Nanoverbindungen von Übergangsmetallen gemäß Anspruch 1 vor; weitere vorteilhafte Ausgestaltungen sind Gegenstand der diesbezüglichen Unteransprüche.

Weiterer Gegenstand der vorliegenden Erfindung ist eine pharmazeutische Zusammensetzung oder ein Arzneimittel, welche bzw. welches mindestens eine Metallcluster-Nanoverbindung enthält, gemäß Anspruch 9; weitere vorteilhafte Ausgestaltungen sind Gegenstand der diesbezüglichen Unteransprüche.

Schließlich ist Gegenstand der vorliegenden Erfindung die Verwendung von lvletallcluster-Nanoverbindungen zur Herstellung eines Arzneimittels gemäß Anspruch 13.

Gegenstand der vorliegenden Erfindung sind somit - gemäß einem **ersten** Aspekt der vorliegenden Erfindung - Metallcluster-Nanoverbindungen von Übergangsmetallen, umfassend einen Metallkern und mindestens einen Liganden, und ihre physiologisch verträglichen Salze zur prophylaktischen und/oder therapeutischen (kurativen) Behandlung von Erkrankungen des menschlichen oder tierischen Körpers, wobei die Metallcluster-Nanoverbindungen die allgemeine Formel

$$[Au_{55} L'_{12} X_6]$$

aufweisen, in der:

- L' einen Liganden aus der Gruppe von Triphenylphosphin und $P(C_6H_5)_2(C_6H_4SO_3H)$, vorzugsweise $P(C_6H_5)_2$(meta-$C_6HSO_3H$), darstellt;
- X ein Halogenatom, vorzugsweise Chlor, darstellt, wobei X in demselben Molekül gleiche oder verschiedene Halogenatome bezeichnen kann.

Unter dem Begriff "Mertallcluster-Nanoverbindungen" im Sinne der vorliegenden Erfindung werden Verbindungen mit lVletall-Metall-Bindungen - in Abgrenzung zu den Mehrkernkomplexen im Wernerschen Sinne - bezeichnet (siehe Römpp Chemielexikon, 10. Auflage, Band 1, 1996, Georg Thieme Verlag, Seiten 773/774, Stichwort: "Cluster-Verbindungen"). Der Begriff der "Cluster" bzw. "Cluster-Verbindungen" wurde im Jahre 1964 von F. A. Cotton eingeführt.

Insbesondere versteht man unter dem Begriff "(Metall-)Cluster" bzw. "(Metall-)Cluster-Verbindung" im Sinne der vorliegenden Erfindung eine Gruppe bzw. einen Kern von 3 oder mehr Übergangsmetallatomen, von denen jedes mit mindestens 2 anderen Atomen der Gruppe bzw. des Kerns chemisch verknüpft, also wenigstens Teils eines Ringes ist, wobei die Gruppe bzw. der Kern von Übergangsmetallen durch geeignete, insbesondere stabilisierende Liganden abgesättigt bzw. umgeben ist. Der Metallkern von Cluster-Verbindungen kann aus Übergangsmetallatomen gleicher (mononukleare Cluster) oder verschiedener (heteronukleare Cluster) Übergangsmetalle bestehen. Als Liganden, die stabilisierend wirken, enthalten solche Verbindungen beispielsweise organische Reste, insbesondere solche mit freien Elelttronenpaaren (z. B. Carbonylreste oder Triphenylphosphinreste).

Der Begriff "(Metall-)Cluster" bzw. "(Metall-)Cluster-Verbindung", wie er erfindungsgemäß verwendet wird,

bezeichnet also die gesamte Verbindung, die aus Metallkern und Liganden besteht.

Bei den Metallcluster-Nanoverbindungen, wie sie erfindungsgemäß verwendet werden, handelt es sich also um Nanopartikel mit mittleren Durchmessern im Bereich von einigen Ångström bis einigen Nanometern, welche aus einem eigentlichen Metallkern bestehen, der insbesondere an seiner Außenschicht mit Liganden umgeben bzw. abgesättigt ist. Daher kann synonym für die Bezeichnung "Metallcluster-Nanoverbindungen" auch die Bezeichnung "Metallnanocluster" verwendet werden.

Solche Metallcluster-Nanoverbindungen von Übergangsmetallen sind an sich aus dem Stand der Technik bekannt (siehe z. B. US-A-5 521 289, US-B1-6 369 206 und US-A-5 360 895). Auch die Verwendung solcher Metallcluster-Nanoverbindungen für wissenschaftliche Zwecke ist bereits bekannt, so z. B. die Verwendung von Grold-Clustern zur Darstellung bzw. Mikroskopierbarkeit von DNA-Molekülen (siehe z. B. Angew. Chem. 2002, 114, Nr. 13, Seiten 2429 bis 2433, Willner et al. "Au-Nanoparticle Nanowires Based on DNA and Polylysine Templates"). Bislang wurde aber keine konkrete therapeutische Anwendung für diese Verbindungen beschrieben. Diese Erkenntnis geht erst auf die Erfinder der vorliegenden Anmeldung zurück.

Physiologisch verträgliche bzw. unbedenkliche Salze der erfindungsgemäß verwendeten Metallcluster-Nanoverbindungen können beispielsweise Salze mit Mineralsäuren, Carbonsäuren oder Sulfonsäuren sein; besonders bevorzugt sind z. B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure. Als physiologisch verträgliche bzw. unbedenkliche Salze können aber beispielsweise auch Salze mit üblichen Basen genannt werden, wie beispielsweise Alkalimetallsalze (z. B. Natrium- oder Kaliumsalze), Erdalkalisalze (z. B. Calcium- oder Magnesiumsalze) oder Ammoniumsalze, abgeleitet von Ammoniak oder organischen Aminen wie beispielsweise Diethylamin, Triethylamin, Ethyldiisopropylamin, Procain, Dibenzylamin, N-Methylmorpholin, Dihydroabiethylamin, 1-Ephenamin oder Methylpiperidin.

Wie zuvor beschrieben können die Metallcluster-Nanoverbindungän von Übergangmetallen einschließlich ihrer physiologisch verträglichen Salze bzw. die Metallkerne solcher Metallcluster-Nanoverbindungen unter bestimmten Voraussetzungen unter physiologischen Bedingungen mit der DNA, vorzugsweise B-DNA, menschlicher oder tierischer Zellen, insbesondere von Tumor- oder Krebszellen, in Wechselwirkung treten, beispielsweise durch Ausbildung physikalischer und/oder chemischer Bindungen. Die B-DNA ist eine spezielle DNA-Konformation, wie sie in wäßrigen Medien, insbesondere unter physiologischen Bedingungen, anzutreffen ist, d. h. die hydratisierte Form.

Um mit der DNA, vorzugsweise B-DNA, menschlicher oder tierischer Zellen, insbesondere von Tumor- oder Krebszellen, in Wechselwirkung treten zu können, muß die mittlere Größe des Metallkerns der Metallcluster-Nanoverbindungen und/oder die Elektronegativität der Metallcluster-Nanoverbindungen und/oder die Stabilisierungsenergie (d. h. die Energie- bzw. Potentialdifferenz zwischen der freien und der DNA-gebundenen Metallcluster-Nanoverbindung) derart ausgewählt werden, daß eine derartige Wechselwirkung möglich ist.

In bezug auf die Größe des Metallkerns der Metallcluster-Nanoverbindungen sollte die Auswahl derart erfolgen, daß die mittlere Größe der Metallkerne der Metallcluster-Nanoverbindungen so ausfällt, daß sie in die großen Furchen *(major grooves)* der DNA-Moleküle, insbesondere von B-DNA, der Tumor- bzw. Krebszellen anbinden können.

Zu diesem Zweck sollte die mittlere Größe der Metallkerne der Metallcluster-Nanoverbindungen höchstens etwa 2,5 nm, insbesondere höchstens etwa 2,0 nm, vorzugsweise höchstens etwa 1,6 nm, besonders bevorzugt höchstens etwa 1,5 nm, ganz besonders bevorzugt etwa 1,4 nm und mindestens etwa 0,5 nm, insbesondere mindestens etwa 0,75 nm, vorzugsweise mindestens etwa 1,0 nm, besonders bevorzugt mindestens etwa 1,3 nm, betragen. Besonders bevorzugt ist, wenn die mittlere Größe der Metallkern der Metallcluster-Nanoverbindungen im Bereich von etwa 1,3 nm bis etwa 1,5 nm liegt.

Die erfindungsgemäß verwendeten Metallcluster-Nanoverbindungen sollten im Hinblick auf die Stabilisierungsenergie derart ausgewählt sein, daß die Stabilisierungsenergie $\Delta E^{stab}$ der Wechselwirkung(en), insbesondere Bindung (en), zwischen der Metallcluster-Nanoverbindung (MCN) und der DNA, insbesondere B-DNA, berechnet als Potentialdifferenz zwischen der Summe aus den Potentialenergien des ligandfreien Metallkerns der Metallcluster-Nanoverbindung $E^{pot}_{MCN}$ und der freien DNA $E^{pot}_{DNA}$ einerseits und der Potentialenergie des resultierenden Komplexes aus dem ligandfreien Metallkern der Metallcluster-Nanoverbindung und DNA $E^{pot}_{MCN-PNA}$ andererseits:

$$\Delta E^{stab} = (E^{pot}_{MCN} + E^{pot}_{DNA}) - E^{pot}_{MCN-DNA}$$

unter Nonnalbedingungen mindestens etwa -400 kJ/mol, insbesondere mindestens etwa -625 kJ/mol, vorzugsweise mindestens etwa -825 kJ/mol, besonders bevorzugt mindestens etwa -1.000 kJ/mol, ganz besonders bevorzugt etwa -1.200 kJ/mol, beträgt. Unter Normalbedingungen werden vorliegend insbesondere eine Temperatur im Bereich von 0 bis 50 °C, insbesondere etwa (20 $\pm$ 5) °C, und ein Druck im Bereich von 10 bis $10^6$ Pa, insbesondere etwa 1,01325

$-10^5$ Pa, verstanden.

Dabei bezieht sich der für die Stabilisierungsenergie $\Delta E^{stab}$ angegebene Wert auf die Reaktion eines ligandfreien Metallkerns mit einem DNA-Molekül. $E^{pot}$MCN bezeichnet die Potentialenergie eines ligandfreien Metallkerns der Metallcluster-Nanoverbindung. d. h. eines "nackten" Metallkerns im (ligand-) freien Zustand, also vor der Anbindung an das DNA-Molekül. $E^{pot}_{DNA}$ bezeichnet die Potentialenergie eines freien DNA-Moleküls, insbesondere B-DNA, d. h. vor Eintritt der Wechselwirkung bzw. Bindung mit dem Metallkern der Metallcluster-Nanoverbindung. $E^{pot}_{MCN\text{-}DNA}$ bezeichnet die Potentialenergie des Produktes bzw. Komplexes aus der Reaktion des einen ligandfreien Metallkerns mit dem einen DNA-Molekül, insbesondere in der B-Konformation.

Wie zuvor erläutert, muß in bezug auf die Elektronegativität der erfindungsgemäß verwendeten Metalleluster-Nanoverbindungen die Auswahl derart erfolgen, daß die Metalleluster-Nanoverbindungen mit der DNA, insbesondere B-DNA, von Tumor- bzw. Krebszellen in Wechselwirkung treten können. Als Maß für die Elektronegativität der jeweiligen Metallcluster-Nanoverbindung kann das Redoxpotential E° des den Metallkern der Metallcluster-Nanoverbindung bildenden Übergangsmetalls in der elektrochemischen Spannungsreihe dienen. Bei den erfindungsgemäß verwendbaren Metallcluster-Nanoverbindungen sollte das Redoxpotential, d. h. das Normalpotential E° des den Metallkern bildenden Übergangsmetalls größer 0 V, insbesondere größer +0,25 V, vorzugsweise größer +0,5 V, bevorzugt größer +0,75 V, besonders bevorzugt größer +1,0 V, sein, jeweils bezogen auf das Redoxpotential der Normalwasserstoffelektrode von 0 V (Nullpunkt). Erfindungsgemäß wird Gold (E° = +1,50 V) als das den Metallkern der jeweiligen Metallcluster-Nanoverbindung bildende Übergangsmetall eingesetzt; Gold ist das elektronegativste aller Metalle. Für weitere Einzelheiten zur elektrochemischen Spannungsreihe und den Redoxpotentialen kann verwiesen werden auf Römpp Chemielexikon, 10. Auflage, Band 5, 1998, Georg Thieme Verlag, Seiten 4162/4163, Stichwort "Spannungsreihe".

Damit eine gute physiologische Wirksamkeit und Anwendbarkeit erreicht wird, sollten die Metallcluster-Nanoverbindungen so ausgewählt werden, daß sie in wäßrigen Medien, insbesondere unter physiologischen Bedingungen, löslich oder zumindest dispergierbar sind, Dies kann insbesondere durch die Auswahl geeigneter Liganden gesteuert werden.

Erfindungsgemäß geeignete Liganden sind beispielsweise organische Reste oder Halogene, vorzugsweise Chlor. Beispiele für erfindungsgemäß geeignete organische Verbindungen sind z. B. Triphenylphosphin und dessen Derivate, insbesondere sulfonierte Derivate (z. B. $P(C_6H_5)_2(C_6H_4SO_3H)$).

Erfindungsgemäß eingesetzte Metallcluster-Nanoverbindungen haben einen Metallkern, der 55 Metallatome umfaßt und eine mittlere Größe von etwa 1,0 nm bis etwa 1,5 nm aufweist. Dabei kann der Metallkern einschließlich Ligand(en) insbesondere mittlere Größen von 2 bis 3 nm, bevorzugt etwa 2,5 nm, aufweisen. Erfindungsgemäß eingesetzte Metallcluster-Nanoverbindungen haben als Metallkern einen $Au_{55}$ -Kern, der von mehreren geeigneten Liganden, wie zuvor definiert, umgeben ist.
Erfindungsgemäß bevorzugt sind die Metallcluster-Nanoverbindungen der Formel

$$[Au_{55}\ \{P(C_6H_5)_2(C_6H_4SO_3H)\}_{12}\ Cl_6]$$

einschließlich ihrer physiologisch verträglichen Salze,

Erfindungsgemäß besonders bevorzugt ist die Metalleluster-Nanoverbindung der Formel

$$[Au_{55}\ \{P(C_6H_5)_2(meta\text{-}C_6H_4SO_3H)\}_{12}\ Cl_6]$$

einschließlich ihrer physiologisch verträglichen Salze.

Die Verbindungen $[Au_{55}\ \{P(C_6H_5)_2(C_6SO_3H)\}_{12}\ Cl_6]$ bzw. $[Au_{55}\ \{P(C_6H_5)_2\ (meta\text{-}C_6H_4SO_3H)\}_{12}\ Cl_6]$ können hergestellt werden durch Ionenaustausch der entsprechenden Natriumsulfonate $[Au_{55}\ P(C_6H_5)_2\ (C_6H_4\ SO_3Na)\}_2Cl_6]$ bzw. $[AU_{55}\ \{P(C_6H_5)_2(meta\text{-}C_6H_4SO_3Na)\}_{12}Cl_6]$ an sauren Ionenaustauschern (Angew. Chem. Int. Ed. Eng. 1995, 34, No. 13/14, Seiten 1442 ff., G. Schmid et al. "First Steps Towards Ordered Monolayers of Ligand-Stabilized Gold Clusters"). Die Natriumsulfonate wiederum werden durch die folgende Phasentransfer-Reaktion erhalten (Polyhedron, Bd.7, Nr, 22/23, 1988, Seiten 2321 bis 2329, G, Schmid "Metal Clusters And Cluster Metals"):

$$\underset{\text{in } CH_2Cl_2}{[Au_{55}\ \{P(C_6H_5)_3\}_{12}\ Cl_6]} + 12\ \underset{\text{in } H_2O}{(C_6H_5)_2P(C_6H_4SO_3Na)}$$

$$\rightarrow \underset{\text{in } H_2O}{[Au_{55}\ \{P(C_6H_5)_2(C_6H_4SO_3Na)\}_{12}\ Cl_6]} + 12\ \underset{\text{in } CH_2Cl_2}{P(C_6H_5)_3}$$

Die Verbindung $[Au_{55}\ \{P(C_6H_5)_3\}_{12}\ Cl_6]$ schließlich kann durch Umsetzung von $[Au\ Cl\ \{P(C_6Hs)_3\}]$ mit Diboran

$B_2H_6$ in warmem Benzol oder Toluol hergestellt werden (Inorganic Syntheses, Bd. 27, Edition A. P. Ginsberg, John Wiley Verlag 1990, Abschnitt 41 "Hexachlorododecakis(triphenylphosphine)-pentapentacontagold", Seiten 214 bis 218). In analoger Weise lassen sich die entsprechenden Rhodium-, Ruthenium- und Palladiumkomplexe herstellen (siehe Inorganic Syntheses, Bd. 27, Edition A. P. Ginsberg, John Wiley Verlag 1990 mit dort zitierter Literatur).

Um eine besonders gute Applizierbarkeit der zuvor beschriebenen Metallcluster-Nanoverbindungen, insbesondere auch unter physiologischen Bedingungen, sicherzustellen, weisen die erfindungsgemäß eingesetzten bzw. ausgewählten Metallcluster-Nanoverbindungen der zuvor beschriebenen Art vorteilhafterweise eine gute Wasserlöslichkeit, insbesondere eine Wasserlöslichkeit von mindestens 0,1 μmol/1, vorzugsweise mindestens 1,0 μmol/1, besonders bevorzugt mindestens 1 mmol/l oder mehr, und bis zu 100 mmol/l und mehr, auf.

Die zuvor beschriebenen Metallcluster-Nanoverbindungen einschließlich ihrer physiologisch verträglichen Salze besitzen ein bislang nicht erkanntes therapeutisches Potential in bezug auf die Behandlung von Erkrankungen des menschlichen oder tierischen Körpers, insbesondere von Tumor- und/oder Krebserkrankungen, hierunter die Behandlung von Primärturmoren, Metastasen und Präkanzerosen (Krebsvorstufen). So eignen sich die zuvor beschriebenen Metallcluster-Nanoverbindungen zur prophylaktischen und therapeutischen bzw. kurativen Behandlung benigner wie maligner Tumore, insbesondere beispielsweise für die Behandlung von Darmkrebs (Kolonkarzinomen), Brustkrebs (Mammakarzinomen), Ovarialkarzinomen, Uteruskarzinomen, Lungenkrebs, Magenkrebs, Leberkrebs, Pankreaskarzinomen, Nierenkrebs, Blasenkrebs, Prostatakrebs, Hodenkrebs, Knochenkrebs, Hautkrebs, Kaposi-Sarkomen, Hirntumoren, Myosarkomen, Neuroblastomen, Lymphomen und Leukämien.

Es wurde gefunden, daß die erfindungsgemäß verwendeten, zuvor beschriebenen Metalleluster-Nanoverbindungen einschließlich ihrer physiologisch verträglichen Salze in der Lage sind, das Zellwachstum bzw. die Zellteilung von Tumor- und Krebszellen zu hemmen bzw, zum Stillstand zu bringen, sogar eine Zerstörung der DNA von Tumor- und Krebszellen zu induzieren.

So wurde gefunden, daß die erfindungsgemäß verwendeten, zuvor beschriebenen Metallcluster-Nanoverbindungen in in-vitro-Studien sich als besonders wirksam auch gegenüber cis-Platin-resistenten Tumoren erwiesen haben. Bei der Behandlung von Tumoren, welche gegenüber cis-Platin nicht resistent sind, wurde eine im Vergleich zum cis-Platin deutlich verbesserte Wirksamkeit gefunden.

Ohne sich auf eine bestimmte Theorie festlegen zu wollen, wird davon ausgegangen, daß die erfindungsgemäß verwendeten Metallcluster-Nanoverbindungen sich in die großen Furchen (*major grooves*) der DNA, insbesondere B-DNA, von Tumor- bzw. Krebszellen einlagern und dort mit der DNA in Wechselwirkung treten können,

Verbindungen mit einem $Au_{55}$ -Kern, insbesondere die Verbindungen $[Au_{55}\{P(C_6H_5)_2(C_6H_4SO_3H)\}_{12}Cl_6]$ bzw. $[Au_{55}\{P(C_6H_5)_2(meta\text{-}C_6H_4SO_3H)\}_{12}Cl_6]$, haben sich dabei als besonders wirksam herausgestellt. Studien der Anmelderin haben ergeben, daß die freie Säure ein noch stärkeres pharmazeutisches Potential bzw. eine bessere Wirksamkeit im Vergleich zum entsprechenden Alkalisulfonat besitzt. Ohne sich auf eine bestimmte Theorie festlegen zu wollen, läßt sich die Wirksamkeit dieser Verbindungen möglicherweise damit erklären, daß sie mit den GCA-Basensequenzen der betreffenden DNA in Wechselwirkung treten.

Fig. 1 zeigt schematisch die Einlagerung von drei Metallkemen von erfindungsgemäß verwendeten Metallcluster-Nanoverbindungen, insbesondere $Au_{55}$ -Kernen, in die großen Furchen (*major grooves*) eines B-DNA-Stranges einer Krebs- bzw. Tumorzelle, wobei in der schematischen Darstellung die Liganden nicht mitdargestellt sind. Auf diese Weise verhindern dann die in den großen Furchen der DNA angeordneten, mit diese in Wechselwirkung getretenen $Au_{55}$ -Kerne eine Teilung der DNA und somit eine Vermehrung der entsprechenden Zelle.

Weiterer Gegenstand der vorliegenden Erfindung - gemäß einem **zweiten** Aspekt der vorliegenden Erfindung - sind pharmazeutische Zusammensetzungen oder Arzneimittel, die mindestens eine wie zuvor beschriebene Metallcluster-Nanoverbindung bzw, ihre physiologisch verträglichen Salze zusammen mit einem pharmazeutisch verträglichen, im wesentlichen nichttoxischen Träger oder Exzipienten enthalten.

Weiterer Gegenstand der vorliegenden Erfindung - gemäß einem **dritten** Aspekt der vorliegenden Erfindung - ist schließlich die Verwendung der zuvor beschriebenen Metallcluster-Nanoverbindungen einschließlich ihrer physiologisch verträglichen Salze zur Herstellung eines Arzneimittels oder einer pharmazeutischen Zusammensetzung zur prophylaktischen und/oder therapeutischen (kurativen) Behandlung von Tumor- und/oder Krebserkrankungen.

Die erfindungsgemäß eingesetzten Metalleluster-Nanoverbindungen bzw. ihre physiologisch verträglichen Salze können gegebenenfalls in Kombination mit einem weiteren pharmazeutischen Wirkstoff, insbesondere einem Chemotherapeutikum und/ oder einem Cytostatikum, entweder als funktionelle Einheit, insbesondere in Form einer Mischung, eines Gemisches oder eines Gemenges, oder aber (räumlich) getrennt voneinander vorliegen, eingesetzt werden.

Die erfindungsgemäß eingesetzten Wirkstoffe bzw. Wirkstoffkombinationen können, je nach Art der zur behandelnden Erkrankungen, systemisch oder aber auch topisch, insbesondere lokal, appliziert werden.

Für die Applikation der erfindungsgemäß eingesetzten Wirkstoffe bzw. Wirkstoffkombinationen kommen alle üblichen Applilcationsformen in Betracht. Beispielsweise kann die Applikation oral, lingual, sublingual, bukkal, rektal oder parenteral (d. h. unter Umgehung des Intestinaltraktes, also intravenös, intraarteriell, intrakardial, intrakutan, subkutan, transdermal, intraperitoneal oder intramuskulär) erfolgen, insbesondere geeignet sind die orale und die intravenöse Applikation; ganz besonders bevorzugt ist die orale Applikation. Weiterhin ist eine topische Anwendung möglich (z. B. zur Behandlung von Melanomen).

Eine besondere Form der topischen Anwendung besteht darin, die Wirkstoffe bzw. Wirkstofflcambinationen in ein Trägersystem, insbesondere ein Drug-Delivery-System, einzubringen und dieses Trägersystem in das Tumor- bzw. Krebsgewebe oder zumindest in die Nähe bzw. Umgebung des Tumor- bzw. Krebsgewebes zu implantieren, wo das Trägersystem dann gezielt am Ort des Tumor- bzw. Krebsgewebes die Wirkstoffe bzw. Wirkstoffkombinationen freisetzt. Auf diese Weise können Nebenwirkungen, wie sie bei der systemischen Applikation auftreten können, vermieden werden, d. h, die Gesamtkörperbelastung kann deutlich reduziert werden. Erfindungsgemäß geeignete, implantierbare Träger- bzw. Drug-Delivery-Systeme sind beispielsweise beschrieben in der auf die Anmelderin selbst zurüelegehenden internationalen Offenlegungsschrift WO 00125841 A1, deren gesamter Inhalt hiermit durch Bezugnahme eingeschlossen ist. Bei dem in der WO 00/25841 A1 beschriebenen Träger- bzw. Drug-Delivery-System kann z. B. die Freisetzung der Wirkstoffe bzw. Wirkstoffkombinationen gezielt gesteuert werden (z. B. durch die Variation der Größe der Öffnungen zur Freisetzung der Wirkstoffe bzw. Wirkstoffkombinationen, durch chemische Modifizierung der Oberfläche etc.).

Für die erfindungsgemäße Anwendung werden die Wirkstoffe bzw. Wirkstoffkombinationen in bekannter Weise in die üblichen Formulierungen überführt, wie z. B. Tabletten, Dragees, Pillen, Granulate, Aerosole, Sirupe, Emulsionen, Suspensionen, Lösungen, Salben, Cremes und Gele aller Art, insbesondere unter Verwendung inerter, im wesentlichen nichttoxischer, pharmazeutisch geeigneter Trägerstoffe oder Lösungsmittel. Hierbei können die erfindungsgemäß eingesetzten Wirkstoffe bzw. Wirkstoffkombinationen jeweils in einer therapeutisch wirksamen Konzentration, insbesondere in Konzentrationen von etwa 0,0001 bis etwa 99 Gew.-%, bevorzugt etwa 0,01 bis etwa 95 Gew,-%, der Gesamtmischung vorhanden sein, d. h. in Mengen, die ausreichend sind, um den angegebenen bzw. gewünschten Dosierungsspielraum zu erreichen. Trotzdem kann es gegebenenfalls erforderlich sein, von den zuvor genannten Mengen abzuweichen, und zwar in Abhängigkeit vom Körpergewicht bzw. von der Art des Applikatiorlsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über einen definierten Zeitraum, z. B. über den Tag, zu verteilen.

Die Formulierungen werden beispielsweise hergestellt durch Verstrecken der Wirkstoffe bzw. Wirkstoffkombinationen mit Lösungsmitteln (z. B. Öle wie Rizinusöl) und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Je nach Applikationsart hat es sich als vorteilhaft erwiesen, die erfindungsgemäß eingesetzten Wirkstoffe bzw. Wirlcstoflicombinationen in Mengen von etwa 0,0001 bis etwa 500 mg/kg Körpergewicht, insbesondere etwa 0,0001 bis etwa 100 mg/kg, vorzugsweise 0,01 bis 50 mg/kg, zur Erzielung wirksamer Ergebnisse zu verabreichen. Trotzdem kann es gegebenenfalls erforderlich sein, von den zuvor genannten Mengen abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese über einen definierten Zeitraum, z, B. über den Tag, zu verteilen, und zwar z. B. in mehreren Einzelgaben oder als Dauerapplikation (z. B. Dauerinfusion), Gleichermaßen ist die Anwendung in einer chronischen Therapie (z. B. in Tablettenform) möglich.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der folgenden Ausführungsbeispiele veranschaulicht, welche die vorliegende Erfindung jedoch keinesfalls beschränken.

## AUSFÜHRUNGSBEISPIELE

### Beispiel 1. A): Herstellung von [$Au_{55}$ {P{$C_6H_5$)s}$_{12}$ $Cl_6$]

Die Verbindung [$Au_{55}$ {$P(C_6H_5)_3$}$_{12}$$Cl_6$] wird gemäß Inorganic Syntheses, Bd. 27, Edition A. P. Ginsberg, John Wiley Verlag 1990, Herstellungsvorschrift Nr. 41, Seiten 214 bis 218 hergestellt. Hierzu wird Aucl[$P(C_6H_5)_3$] mit Diboran $B_2H_6$ in warmem Benzol oder Toluol umgesetzt. Das Diboran $B_2H_6$ selbst kann gemäß der folgenden Reaktionsgleichung hergestellt werden:

$$3\ NaBH4 + 4\ BF_3\ .\ O(C_2H_5)_2 \rightarrow 3\ NaBF_4 + 2\ B_2H_6 + 4\ (C_2H_5)_2O$$

Die Verbindung [$Au_{55}$ {$P(C_6H_3)_3$}$_{12}$$Cl_6$] ist ein dunkelbraunes Pulver, das sich in Dichlormethan und Pyridin lösen läßt.

**Beispiel 1. B): Herstellung von [$Au_{55}$ {$P(C_6H_5)_2(C_6H_4SO_3Na)$}$_{12}$$Cl_6$]**

Die Herstellung von [$Au_{55}$ {$P(C_6H_5)_2(C_6H_4SO_3Na)$}$_{12}$$Cl_6$] erfolgt durch Umsetzung der in Beispiel 1. A) hergestellten Verbindung mit [$P(C_6H_5)_3$] in einer Phasentransfer-Reaktion gemäß der folgenden Reaktionsgleichung (siehe Polyhedron, Bd. 7, Nr. 22/23, 1988, Seiten 2321 bis 2329):

$$\underset{\text{in } CH_2Cl_2}{[Au_{55}\{P(C_6H_5)_3\}_{12}Cl_6]} + \underset{\text{in } H_2O}{12\,(C_6H_5)_2P(C_6H_4SO_3Na)}$$

$$\rightarrow \underset{\text{in } H_2O}{[Au_{55}\{P(C_6H_5)_2(C_6H_4SO_3Na)\}_{12}Cl_6]} + \underset{\text{in } CH_2Cl_2}{12\,P(C_6H_5)_3}$$

**Beispiel 1. C): Herstellung Von [$Au_{55}$ {$P(C_6H_5)_2(C_6H_4SO_3H)$}$_{12}$$Cl_6$]**

Die freie Sulfonsäure [$Au_{55}$ {$P(C_6H_5)_2(C_6H_4SO_3H)$}$_{12}$$Cl_6$] kann ausgehend von der in Beispiel 1.B) hergestellten $Au_{55}$-Clusterverbindung hergestellt werden, indem letztere über einen sauren Ionenaustauscher gegeben wird (Angew. Chem. Int. Ed. Engl. 1995, 34, No. 13/14, Seiten 1442 ff.). Die freie Säure erweist sich in den im folgenden beschriebenen in-vitro-Zelltoxizitätsmessungen als besonders wirksam in bezug auf Tumor- bzw. Krebszellen.

**Beispiel 2: In-vitro-Zelltoxhitätsmessungen**

Die in-vitro-Cytotoxizitätseigenschaften der in Beispiel 1. C) hergestellten Gold-55-Partikel ($Au_{55}$) wurden an HeLa-Krebszellen, sowie an MOR/P- und MOR/CPR-Lungenkrebszellen vorgenommen. MOR/P-Zellcn reagieren empfmdlich auf cis-Platin, während MOR/CPR-Zellen gegenüber cis-Platin resistent sind.

Die HeLa-Zellen wurden auf einem DMEM-Medium bei 37°C in einer 5%igen $CO_2$-Atmosphä gezüchtet. Das Medium war mit 10%igem FCS-Serum sowie Antibiotika versetzt. Zweimal wöchentlich wurden Tochterkulturen angelegt. Die MOR/P- und MOR/CPR-Zellen wurden auf einem RPMI 1640-Medium bei 37 °C in einer 5%igen $CO_2$-Atmosphäre gezüchtet. Das Medium war ebenfalls mit 10%igem FCS-Serum sowie Antibiotika versetzt. Zweimal wöchentlich wurden auch hier Tochterkulturen angelegt.

Die Bestimmungen der in-vitro-Cytotoxizität der $Au_{55}$-Partikel wurden mit 96-Well-Mikrotiterplatten und einem MTT-Kolorimetrie-Assay (CellTiter 96® Aqueous One Solution Cell Proliferation Assay, Promega) auf folgende Weise durchgeführt:

Kulturen einer jeden Zell-Linie mit einer Konzentration von $1x10^5$ Zellen/mL wurden auf die Mikrotiterplatten gegeben und 72 Stunden in den oben beschriebenen Medien bei 37 °C in einer 5%igen $CO_2$-Atmosphäre gezüchtet. Anschließend wurden $Au_{55}$ -Partikel jeweils in 50 $\mu$L der RPMI- und DMEM-Medien gelöst und so zugegeben, daß folgende $Au_{55}$ -Konzentrationen von 0.5, 0.75, 1.0, 3.0, 6.0, 10,0 und 50.0 $\mu$M resultierten. Ein Ansatz von Kontrollzellen ohne Gold-5 5-Partikel enthielt 50 $\mu$L des DMEM- bzw. RPMI Medium. Die Milcrotiterplatten wurden 15 Stunden bei 37°C in einer 5%igen $CO_2$-Atmosphäre inkubiert.

Nach der Inkubation der Kebszellen mit $Au_{55}$-Partikel wurden 40 $\mu$L des MTT-Reagenzes zu jedem Well jeder Mikrotiterplatte zugesetzt. Anschliessend wurde 4 Stunden bei 37 °C inkubiert.

Die Absorption bei 490 nm wurde für jeden Well jeder Mikrotiterplatte durch einen 96-Well-Mikrotitezplatten-Reader gemessen. Die gemessene Adsorption bei 490 nm wurde gegen die $Au_{55}$ -Partikelkonzentration aufgetragen, und der $IG_{50}$-Wert wurde bestimmt.

**Abb. 1** zeigt das Profil der Sensitivität der HeLa-Krebszellen gegenüber den in Beispiel 1. C) hergestellten $Au_{55}$ -Partikeln. Der Graph zeigt den Verlauf der Absorption bei 490 nm nach der Inkubation in Abhängigkeit der Zunahme der Konzentration der $Au_{55}$ -Partikel. Das Experiment wurde durch dreimalige Wiederholung bestätigt. Der $IC_{50}$-Wert (50 % der Zellen sind inaktiv) für diese Zellinie wurde bei einer $Au_{55}$ -Konzentration von 5.0 $\mu$M bestimmt. Über den $IC_{50}$-Wert für cis-Platin und HeLa-Krebszellen ist bisher nichts bekannt.

**Abb. 2** zeigt das Profil der Sensitivität der cis-Platin empfindlichen humanen MOR/P-Lungenkrebszellen gegenüber den in Beispiel 1. C) hergestellten $Au_{55}$ -Partikeln. Der Graph zeigt den Verlauf der Absorption bei 490 nm nach der Inkubation und den Vergleich mit den Kontrollzellen in Abhängigkeit der Zunahme der Konzentration der $Au_{55}$ -Partikel. Die einzelnen Linien kennzeichnen unabhängige Experiment, die jeweils dreimal wiederholt wurden. Der

$IC_{50}$-Wert wurde für diese Zellinie bei einer $Au_{55}$-Konzentration von 2,l ±0.07 μM bestimmt. Der $TC_{50}$-Wert für cis-Platin ist für diese Zell-Linie bei 3.1 ± 0.3 μM.

**Abb. 3** zeigt das Profil der Sensitivität der cis-Platin-resistenten humanen MOR/CPR-Lungenkrebszellen gegenüber den in Beispiel 1. C) hergestellten $Au_{55}$ -Partikeln, Der Graph zeigt den Verlauf der Absorption bei 490 nm nach der Inkubation und den Vergleich mit den Kontrollzellen in Abhängigkeit der Zunahme der Konzentration der $Au_{55}$ -Partikel. Die einzelnen Linien kennzeichnen unabhängige Experimente, die jeweils dreimal wiederholt wurden, Der $IC_{50}$-Wert wurde für diese Zellinie bei einer $Au_{55}$-Konzentration von (2,0 ± 0.21) μM bestimmt. Der $IC_{50}$-Wert für cis-Platin liegt für diese Zellinie bei (7.1 ± 1.2) μM.

**Beispiel 3: DNA-Spaltung durch Restriktionsendonukleasen in Gegenwart der in Beispiel 1. C) hergestellten $Au_{55}$-Pardkel**

Es ist bekannt, daß Restriktionsenzyme Doppelstrang-Desoxyribonukleasen an spezifischen Basensequenzen spalten. Es wurden Restriktionsendonukleasen zur DNA-Spaltung verwendet um zu untersuchen, ob die $Au_{55}$ -Partikel mit spezifischen Nukleotiden (Basen) bevorzugt in Wechselwirkung treten. Hierbei wurden die folgenden Enzyme verwendet: Sma 1 (Roche), Hind III (Gibco), Pst I (Gibco) and Sal I (Gibco) benutzt. Diese Enzyme spalten DNA an jeweils unterschiedlichen Stellen (Basensequenzen).

Die DNA-Spaltung durch die verschiedenen Restriktionsendonulcleasen wurde in einem Volumen von 30 μL nach folgendem Prozeß bestimmt: Nach einer Vorinkubation von 15 Stunden bei Raumtemperatur wurden 0.1 μg/μL Plasmid-DNA (pcDNA3. 1/*myc*-His° (-) B, Invitrogen) mit den $Au_{55}$ partikeln zu einer Endkonzentration von 5 μM der $Au_{55}$ -Partikel versetzt. Danach wurden 20 Einheiten/μL des jeweiligen Enzyms und 6 μL einer entsprechenden Enzym-Pufferlösung zugegeben.

Die DNA-Spaltung wurde für Hind III, Pst I and Sal I bei 37 °C und für Sma I bei 25 °C zwei Stunden lang durchgerührt. Der Spaltungsprozeß wurde durch Hitzeinaktivierung, d. h. bei einer Inkubation der Reaktionslösung bei einer Temperatur von 65 °C und einer Wirkzeit von 20 Minuten und nachfolgend bei -20 °C und 10 Minuten gestoppt. Die DNA-Spaltung wurde durch Gel-Elektrophorese sichtbar gemacht. Die Ergebnisse der Untersuchungen sind in der folgende Tab. 1 zusammengestellt:

**Tab. 1:** $Au_{55}$ -behandelte DNA-Spaltung durch Restriktionsenzyme.

| | **Sma I** | **Hind III** | **Pst I** | **Sal I** |
|---|---|---|---|---|
| | CCC↓GGG | A↓AGCTT | CTGCA↓G | G↓ TCGAC |
| **$Au_{55}$ 5 M** | +/- 50% | +/- 50% | +/- 90% | +/- 50% |

Deaktivierung ist durch +/- und einen entsprechenden Prozentwert gekennzeichnet. Es ist offensichtlich, daß die $Au_{55}$-Partikel hauptsächlich das Pst I-Restrilctionsenzym bei der Spaltung der CTGCAG-Basensequenz hemmen. Es kann somit geschlossen werden, daß die $Au_{55}$-Partikel bevorzugt mit der GCA-Basensequenz in Wechselwirkung treten.

**Beispiel 4: Untersuchung des Antitumorpotentials der $Au_{55}$-Clusterwerbindung $Au_{55}(Ph_2PC_6HSO_3H)_{12}Cl_6$ (mit Ph = Phenyl)**

Im vorliegenden Ausftihrungsbeispiel wurde das Antitumorpotential der Verbindung $Au_{55}(Ph_2PC_6H_4O_3H)_{12}Cl_6$, im folgenden bisweilen nur als [$Au_{55}$] bezeichnet, gegenüber einer Reihe humaner Krebszell-Linien untersucht. Die $Au_{55}$-Cluster bestehen außer einem Kern aus 55 Goldatomen aus einer Hülle von 12 wasserlöslichen, monosulfonierten Triphenylphosphan-Molelkülen und 6 Chloratomen (Fig. 2, welche das Modell eines $Au_{55}(PPh_3)_{12}Cl_6$-Clusters darstellt).

Die in-vitro-Cytotoxizität wurde mittels des MTT-Tests (Promega) untersucht, einer kolorimetrischen Methode, bei welcher eine Tetrazolium-basierte Verbindung durch lebende Zellen zu Formazan reduziert wird. Die Menge gebildeten Formazans ist direkt proportional zur Anzahl der lebenden Zellen in der Kultur. Jede Zell-Linie wurde vor der Zugaben der Medikamente in Mikroliter-Schälchen 48 Stunden lang inkubiert. Cisplatin oder $Au_{55}$ wurde zugegeben und 72 bzw. 24 Stunden inkubiert. Danach wurden die MTT-Tests gemäß den Angaben von Promega durchgeführt.

**Abb. 4** zeigt einen typischen Graphen aus einem MTT-Test. Man erkennt, daß mit steigender Konzentration von [$Au_{55}$] die Absorption durch Formazan und hiermit die Lebensdauer der Zellen abnimmt. Ebenso wurde untersucht, ob die Ligandmoleküle selbst Einfluß auf die Lebensdauer der Zellen haben. Dies ist für den untersuchten Fall der Tumorzell-Linie MOR/CPR nicht erkennbar (**Abb. 5**). Im einzelnen:

**Abb. 4** betrifft in-vitro-Cytotoxizitätstests an cisplatinresistenten Lungentumorzellen MOR/CPR, 24 Stunden

mit unterschiedlichen Konzentrationen an $Au_{55}(Ph_2PC_6H_4SO_3H)_{12}Cl_6$ [$Au_{55}$] inkubiert. Jeder Punkt repräsentiert 3 unabhängig voneinander durchgeführte Experimente, jeweils dreimal wiederholt,

**Abb. 5** betrifft in-vitro-Cytotoxizitätstests an cisplatinresistenten Lungentumorzellen MOR/CPR, 24 Stunden mit unterschiedlichen Konzentrationen von freiem Liganden $Ph_2PC_6H_4SO_3H$ inkubiert. Jeder Punkt repräsentiert 3 unabhängige Versuche, durch jeweilige Dreifachbestimmung wiederholt.

Allgemein wurde für die untersuchten Zell-Linien gefunden, daß [$Au_{55}$] eine schnellere und größere Cytotoxizität als Cisplatin aufweist, wie aus den $IC_{50}$-Werten in Tab. 2 ersichtlich. Die einzigen, bisher getesteten gesunden Zellen, nämlich von MC3, reagieren charakteristisch schwächer auf [$Au_{55}$] als die Knochentumorzellen U2OS, Daraus folgt, daß [$Au_{55}$] gegenüber gesunden Zellen weniger toxisch ist als für Tumorzellen. Versuche mit gesunden und Tumorhautzellen (Melanom) zeigen die gleiche Tendenz. Bemerkenswert ist ferner die Tatsache, daß metastatische Melanom-Zellen resistent gegenüber Cisplatin, aber äußerst empfindlich gegenüber [$Au_{55}$] sind, Somit eröffnet sich die Möglichkeit, [$Au_{55}$] besonders in Fällen anzuwenden, in denen eine Cis-platin-Resistenz auftritt.

Die folgende Tab. 2 zeigt die Inhibitionslconzentrationen von Cisplatin- und [$Au_{55}$]-Inkubationen mit verschiedenen Zell-Linien über 72 bzw. 24 Stunden. Die $IC_{50}$-Werte wurden aus den Graphen berechnet, die aus den in-vitro-Cytotoxizitätstests MTT erhalten worden sind. Jedes Experiment wurde dreimal unabhängig voneinander durch jeweilige Dreifachbestimmung wiederholt.

**Tab. 2**

| Zell-Linie | | $IC_{50}$Cisplatin 72h | $IC_{50}$ [$Au_{55}$] 24h |
|---|---|---|---|
| MC3 | Normale Knochenzellen | 26.1 ± 1.27 μM | 1.65 ± 0.14 μM |
| U2OS | Osteosarkom | 11.17 ± 2.02 μM | 0.64 ± 0.04 μM |
| MOR/P | Lungenkrebszellen cisplatinsensitiv | 3.30 ± 0.3 μM | 2.10 ± 0.10 μM |
| MOR/CPR | Lungenkrebszellen cisplatinresistent | 7.10 ± 1.2 μM | 2.50 ± 0.10 μM |
| BLM | Metastatisches Melanom | 54.70± 7.60 μM | 0.30±0,10 μM |
| MV3 | Metastatisches Melanom | >50 μM | 0.24 ± 0.02 μM |
| HeLa | Cervical cancer cells | 7.93 ± 0.95 μM | 2.29 ± 0.10 μM |
| Hek | Nierenkrebszellen transfiziert mit Adenovirus | 20.13 ± 6.0 μM | 0.63 ± 0.02 μM |

**Patentansprüche**

1. Metallcluster-Nanoverbindungen von Übergangsmetallen, umfassend einen Metallkern und mindestens einen Liganden, und ihre physiologisch verträglichen Salze zur prophylaktischen und/oder therapeutischen (kurativen) Behandlung von Erkrankungen des menschlichen oder tierischen Körpers, wobei die Metallcluster-Nanoverbindungen die allgemeine Formel

$$[Au_{55}\,L'_{12}X_6]$$

aufweisen, in der:

- L' einen Liganden aus der Gruppe von Triphenylphosphin und $P(C_6H_5)_2(C_6H_4SO_3H)$, vorzugsweise $P(C_6H_5)$ (meta-$C_6H_4SO_3H$), darstellt;
- X ein Halogenatom, vorzugsweise Chlor, darstellt, wobei X in demselben Molekül gleiche oder verschiedene Halogenatome bezeichnen kann.

2. Metallcluster-Nanoverbindungen nach Anspruch 1, wobei die Metallcluster-Nanoverbindungen die Formel

$$[Au_{55}\,\{P(C_6H_5)_2(C_6H_4SO_3H)\}_{12}Cl_6]$$

aufweisen.

3. Metallcluster-Nanoverbindungen nach Anspruch 1 oder 2, wobei die Metallcluster-Nanoverbindungen die Formel

$$[Au_{55}\,\{P(C_6H_5)_2(\text{meta-}C_6H_4SO_{3H})\}_{12}Cl_6]$$

aufweisen.

4. Metallcluster-Nanoverbindungen nach einem der Ansprüche 1 bis 3, **gekennzeichnet durch** eine gute Wasserlöslichkeit, insbesondere eine Wasserlöslichkeit von mindestens 0,1 μmol/1, vorzugsweise mindestens 1,0 μmol/1, besonders bevorzugt mindestens 1 mmol/l oder mehr, und bis zu 100 mmol/l und mehr.

5. Metallcluster-Nanoverbindungen nach einem der Ansprüche 1 bis 4 zur prophylaktischen und/oder therapeutischen (kurativen) Behandlung von Tumor- und/oder Krebserkrankungen des menschlichen oder tierischen Körpers, insbesondere von Primärtumoren und/oder Metastasen und/oder Präkanzerosen (Krebsvorstufen), insbesondere zur prophylaktischen und/oder therapeutischen (kurativen) Behandlung von Darmkrebs (Kolonkarzinomen), Brustkrebs (Mammakarzinomen), Ovarialkarzinomen, Uteruskarzinomen, Lungenkrebs, Magenkrebs, Leberkrebs, Pankreaskarzinomen, Nierenkrebs, Blasenkrebs, Prostatakrebs, Hodenkrebs, Knochenkrebs, Hautkrebs, Kaposi-Sarkomen, Hirntumoren, Myosarkomen, Neuroblastomen, Lymphomen und Leukämien.

6. Metallcluster-Nanoverbindungen nach einem der Ansprüche 1 bis 5 zur prophylaktischen und/oder therapeutischen (kurativen) Behandlung benigner und maligner Tumore.

7. Metallcluster-Nanoverbindungen nach einem der Ansprüche 1 bis 6, wobei die Metallcluster-Nanoverbindungen das Zellwachstum und/oder die Zellteilung von Tumor- und/oder Krebszellen hemmen und/oder zum Stillstand bringen und/oder wobei die Metallcluster-Nanoverbindungen eine Zerstörung der DNA von Tumor- und/oder Krebszellen induzieren.

8. Metallcluster-Nanoverbindungen nach einem der Ansprüche 1 bis 7, wobei die Metallcluster-Nanoverbindungen systemisch und/oder topisch applizierbar sind.

9. Pharmazeutische Zusammensetzung oder Arzneimittel, enthaltend mindestens eine Metallcluster-Nanoverbindung, wie in den Ansprüchen 1 bis 8 definiert, und/oder ihre physiologisch verträglichen Salze zusammen mit einem pharmazeutisch verträglichen, im wesentlichen nichttoxischen Träger oder Exzipienten.

10. Pharmazeutische Zusammensetzung oder Arzneimittel nach Anspruch 9, enthaltend die mindestens eine Metallcluster-Nanoverbindung, wie in den Ansprüchen 1 bis 8 definiert, und/oder ihre physiologisch verträglichen Salze in therapeutisch wirksamen Mengen.

11. Pharmazeutische Zusammensetzung oder Arzneimittel nach Anspruch 9 oder 10, umfassend außerdem mindestens einen weiteren pharmazeutischen Wirkstoff, insbesondere ein Chemotherapeutikum und/oder ein Cytostatikum, entweder als funktionelle Einheit, insbesondere in Form einer Mischung, eines Gemisches oder eines Gemenges, oder aber (räumlich) getrennt voneinander vorliegend.

12. Pharmazeutische Zusammensetzung oder Arzneimittel nach einem der Ansprüche 9 bis 11 zur systemischen und/oder topischen Anwendung.

13. Verwendung von Metallcluster-Nanoverbindungen, wie in den Ansprüchen 1 bis 8 definiert, und/oder ihrer physiologisch verträglichen Salze zur Herstellung eines Arzneimittels oder einer pharmazeutischen Zusammensetzung zur prophylaktischen und/oder therapeutischen (kurativen) Behandlung von Tumor- und/oder Krebserkrankungen.

## Claims

1. Metal cluster nanocompounds of transition metals, comprising a metal core and at least one ligand, and physiologically tolerated salts thereof for the prophylactic and/or therapeutic (curative) treatment of disorders of the human or animal body, the metal cluster nanocompounds having the general formula

$$[Au_{55} L'_{12}X_6]$$

where:

- L' is a ligand from the group consisting of triphenylphosphine and $P(C_6H_5)_2(C_6H_4SO_3H)$, preferably $P(C_6H_5)_2$ (meta-$C_6H_4SO_3H$);
- X is a halogen atom, preferably chlorine, it being possible for X to denote identical or different halogen atoms in the same molecule.

**2.** Metal cluster nanocompounds according to Claim 1, the metal cluster nanocompounds having the formula

$$[Au_{55}\{P(C_6H_5)_2(C_6H_4SO_3H)\}_{12}Cl_6].$$

**3.** Metal cluster nanocompounds according to Claim 1 or 2, the metal cluster nanocompounds having the formula

$$[Au_{55}\{P(C_6H_5)_2(\text{meta-}C_6H_4SO_3H)\}_{12}Cl_6].$$

**4.** Metal cluster nanocompounds according to any of Claims 1 to 3, **characterized by** good water solubility, in particular a water solubility of at least 0.1 μmol/1, preferably at least 1.0 μmol/1, particularly preferably at least 1 mmol/l or more, and up to 100 mmol/l and more.

**5.** Metal cluster nanocompounds according to any of Claims 1 to 4 for the prophylactic and/or therapeutic (curative) treatment of neoplastic and/or cancerous disorders of the human or animal body, in particular of primary tumors and/or metastases and/or precancerous diseases (pre-cancer stages), in particular for the prophylactic and/or therapeutic (curative) treatment of colon cancer (colon carcinomas), breast cancer (mamma carcinomas), ovarian carcinomas, carcinomas of the uterus, lung cancer, stomach cancer, liver cancer, carcinomas of the pancreas, kidney cancer, bladder cancer, prostate cancer, testicular cancer, bone cancer, skin cancer, Kaposi sarcomas, brain tumors, myosarcomas, neuroblastomas, lymphomas and leukemias.

**6.** Metal cluster nanocompounds according to any of Claims 1 to 5 for the prophylactic and/or therapeutic (curative) treatment of benign and malignant tumors.

**7.** Metal cluster nanocompounds according to any of Claims 1 to 6, the metal cluster nanocompounds inhibiting and/or stopping cell growth and/or cell division of tumor and/or cancer cells and/or the metal cluster nanocompounds inducing destruction of tumor and/or cancer cell DNA.

**8.** Metal cluster nanocompounds according to any of Claims 1 to 7, it being possible to administer the metal cluster nanocompounds systemically and/or topically.

**9.** Pharmaceutical composition or medicament, comprising at least one metal cluster nanocompound as defined in Claims 1 to 8 and/or physiologically tolerated salts thereof, together with a pharmaceutically tolerated, essentially nontoxic carrier or excipient.

**10.** Pharmaceutical composition or medicament according to Claim 9, comprising the at least one metal cluster nanocompound as defined in Claims 1 to 8 and/or physiologically tolerated salts thereof in therapeutically active amounts.

**11.** Pharmaceutical composition or medicament according to Claim 9 or 10, furthermore comprising at least one further pharmaceutical active compound, in particular a chemotherapeutic and/or a cytostatic agent, present either as a functional unit, in particular in the form of a blend, a mixture or a batch, or else (spatially) separated from one another.

**12.** Pharmaceutical composition or medicament according to any of Claims 9 to 11 for systemic and/or topical application.

**13.** Use of metal cluster nanocompounds as defined in Claims 1 to 8 and/or of physiologically tolerated salts thereof for preparing a medicament or a pharmaceutical composition for the prophylactic and/or therapeutic (curative) treatment of neoplastic and/or cancerous diseases.

**Revendications**

**1.** Nanocomposés à clusters métalliques de métaux de transition, comprenant un noyau métallique et au moins un ligand, et leurs sels physiologiquement acceptables, pour le traitement prophylactique et/ou thérapeutique (curatif) de maladies du corps humain ou animal, les nanocomposés à clusters métalliques présentant la formule générale

$$[Au_{55}L'_{12}X_6]$$

dans laquelle:

• L' représente un ligand choisi dans le groupe constitué par la triphénylphosphine et $P(C_6H_5)_2(C_6H_4SO_3H)$, de préférence $P(C_6H_5)_2$(méta-$C_6H_4SO_3H$);
• X représente un atome d'halogène, de préférence de chlore, X pouvant désigner dans la même molécule des atomes d'halogène identiques ou différents.

**2.** Nanocomposés à clusters métalliques selon la revendication 1, les nanocomposés à clusters métalliques présentant la formule

$$[Au_{55}\{P(C_6H(C_6H_4SO_3H)\}_{12}Cl_6].$$

**3.** Nanocomposés à clusters métalliques selon la revendication 1 ou 2, les nanocomposés à clusters métalliques présentant la formule

$$[Au_{55}\{P(C_6H_5)_2(\text{méta-}C_6H_4SO_3H)\}_{12}Cl_6].$$

**4.** Nanocomposés à clusters métalliques selon l'une quelconque des revendications 1 à 3, **caractérisés par** une bonne solubilité dans l'eau, en particulier une solubilité dans l'eau d'au moins 0,1 µmole/l, de préférence d'au moins 1,0 µmole/1, de façon particulièrement préférée d'au moins 1 mmole/l ou plus, et de jusqu'à 100 mmoles/l et plus.

**5.** Nanocomposés à clusters métalliques selon l'une quelconque des revendications 1 à 4, pour le traitement prophylactique et/ou thérapeutique (curatif) de maladies tumorales et/ou cancéreuses du corps humain ou animal, en particulier de tumeurs primaires et/ou de métastases et/ou de maladies précancéreuses (états précancéreux), en particulier pour le traitement prophylactique et/ou thérapeutique (curatif) du cancer de l'intestin (carcinomes du côlon), du cancer du sein (carcinomes mammaires), de carcinomes ovariens, de carcinomes de l'utérus, du cancer du poumon, du cancer de l'estomac, du cancer du foie, de carcinomes pancréatiques, du cancer du rein, du cancer de la vessie, du cancer de la prostate, du cancer du testicule, du cancer des os, du cancer de la peau, de sarcomes de Kaposi, de tumeurs du cerveau, de myosarcomes, de neuroblastomes, de lymphomes et de leucémies.

**6.** Nanocomposés à clusters métalliques selon l'une quelconque des revendications 1 à 5, pour le traitement prophylactique et/ou thérapeutique (curatif) de tumeurs bénignes et de tumeurs malignes.

**7.** Nanocomposés à clusters métalliques selon l'une quelconque des revendications 1 à 6, les nanocomposés à clusters métalliques inhibant et/ou stoppant la croissance cellulaire et/ou la division cellulaire de cellules tumorales et/ou cancéreuses et/ou les nanocomposés à clusters métalliques induisant une destruction de l'ADN de cellules tumorales et/ou cancéreuses.

**8.** Nanocomposés à clusters métalliques selon l'une quelconque des revendications 1 à 7, les nanocomposés à clusters métalliques pouvant être administrés par voie systémique et/ou topique.

**9.** Composition pharmaceutique ou médicament, contenant au moins un nanocomposé à clusters métalliques tel que défini dans les revendications 1 à 8, et/ou ses sels physiologiquement acceptables, conjointement avec un véhicule ou excipient essentiellement non toxique, pharmaceutiquement acceptable.

**10.** Composition pharmaceutique ou médicament selon la revendication 9, contenant au moins un nanocomposé à clusters métalliques, tel que défini dans les revendications 1 à 8, et/ou ses sels physiologiquement acceptables, en quantités thérapeutiquement efficaces.

**11.** Composition pharmaceutique ou médicament selon la revendication 9 ou 10, comprenant en outre au moins une autre substance active pharmaceutique, en particulier un agent chimiothérapeutique et/ou un agent cytostatique, se trouvant soit sous forme d'unité fonctionnelle, en particulier sous forme d'une composition, d'un mélange ou d'un conglomérat, soit séparée (spatialement) de l'autre composant.

**12.** Composition pharmaceutique ou médicament selon l'une quelconque des revendications 9 à 11, pour l'administration systémique et/ou topique.

**13.** Utilisation de nanocomposés à clusters métalliques, tels que définis dans les revendications 1 à 8, et/ou de leurs sels physiologiquement acceptables, pour la fabrication d'un médicament ou d'une composition pharmaceutique destinés au traitement prophylactique et/ou thérapeutique (curatif) de maladies tumorales et/ou cancéreuses.

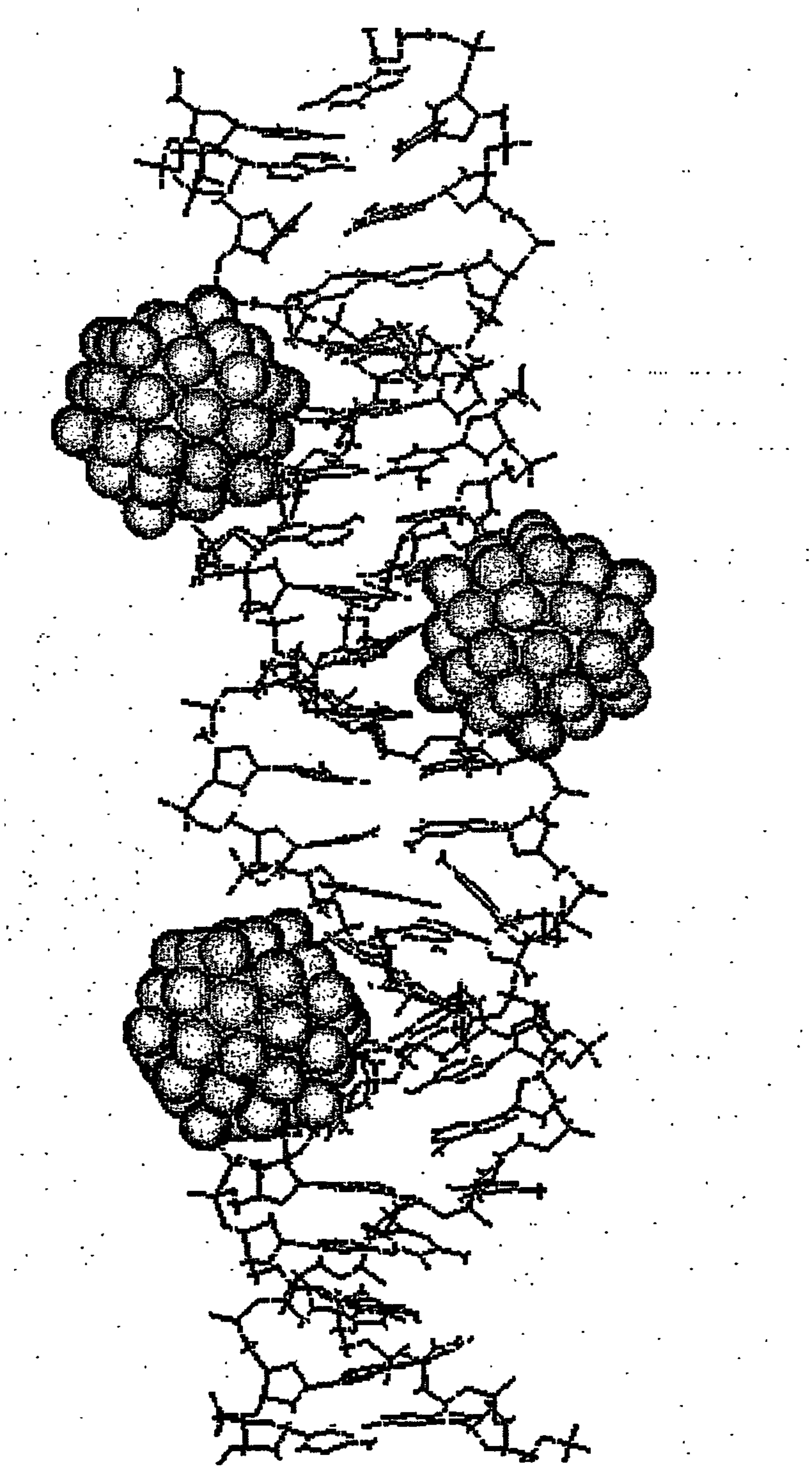

Fig. 1

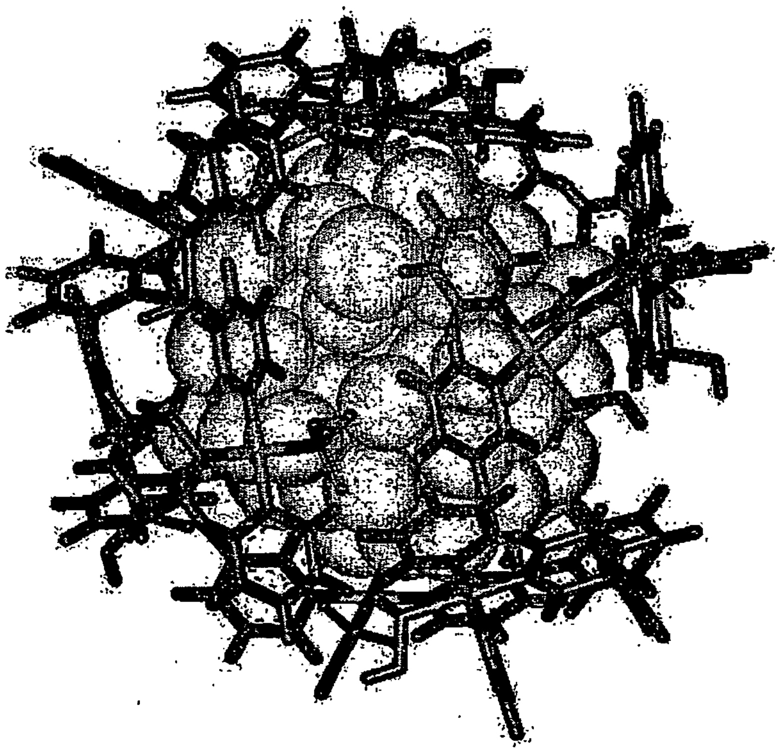

Fig. 2

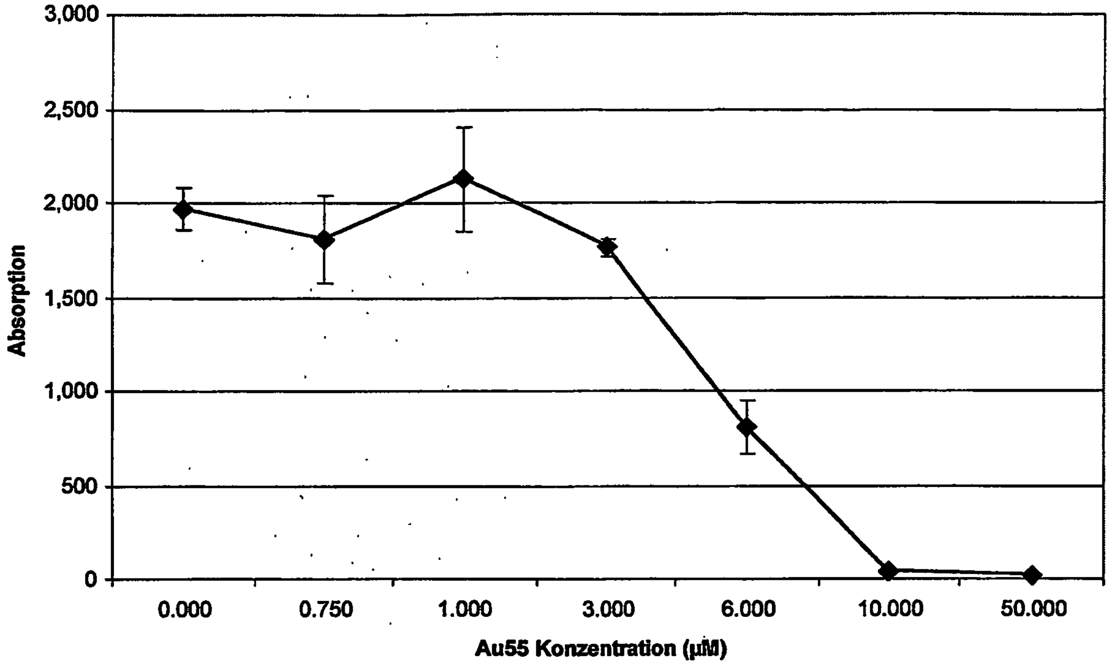
3,000
2,500
2,000
1,500
1,000
500
0
Absorption
0.000
0.750
1.000
3.000
6.000
10.000
50.000
Au55 Konzentration (µM)

FIG. 3

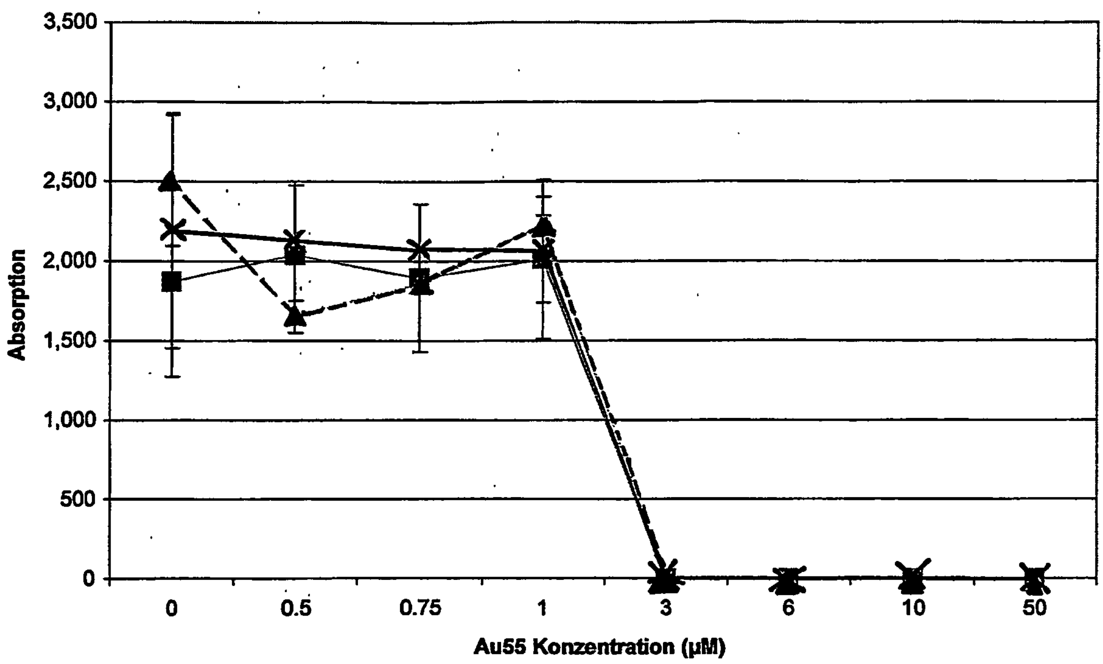
3,500
3,000
2,500
2,000
1,500
1,000
500
0
Absorption
0
0.5
0.75
1
3
6
10
50
Au55 Konzentration (µM)

FIG. 4

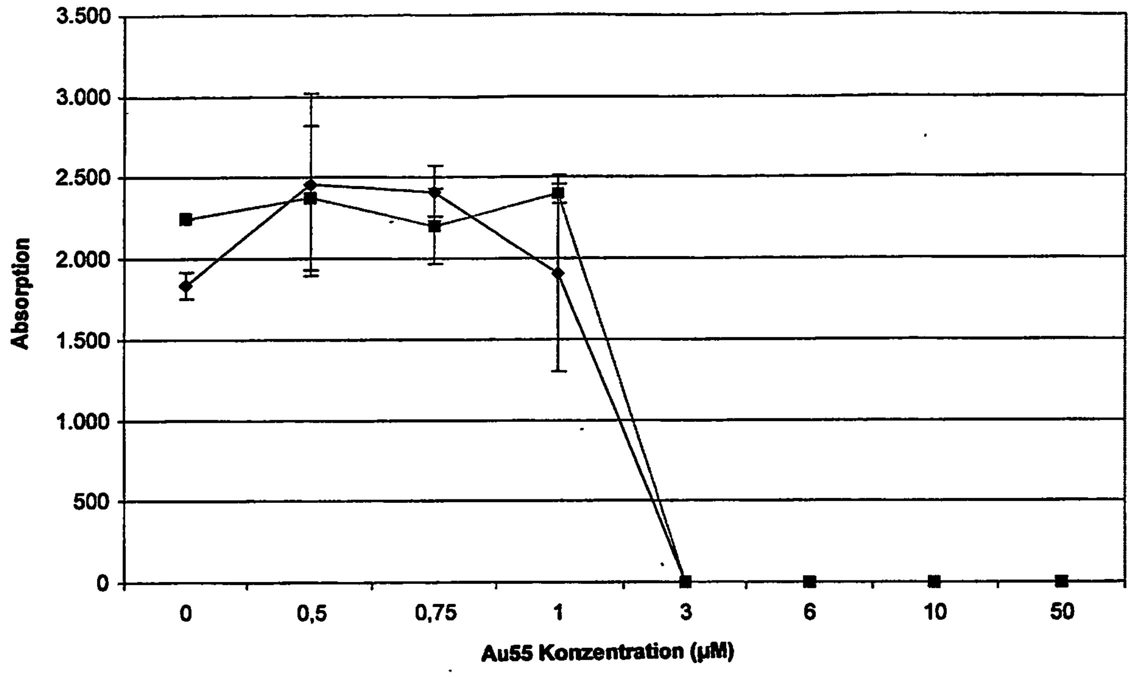
3.500
3.000
2.500
2.000
1.500
1.000
500
0
Absorption
0
0,5
0,75
1
3
6
10
50
Au55 Konzentration (µM)

FIG. 5

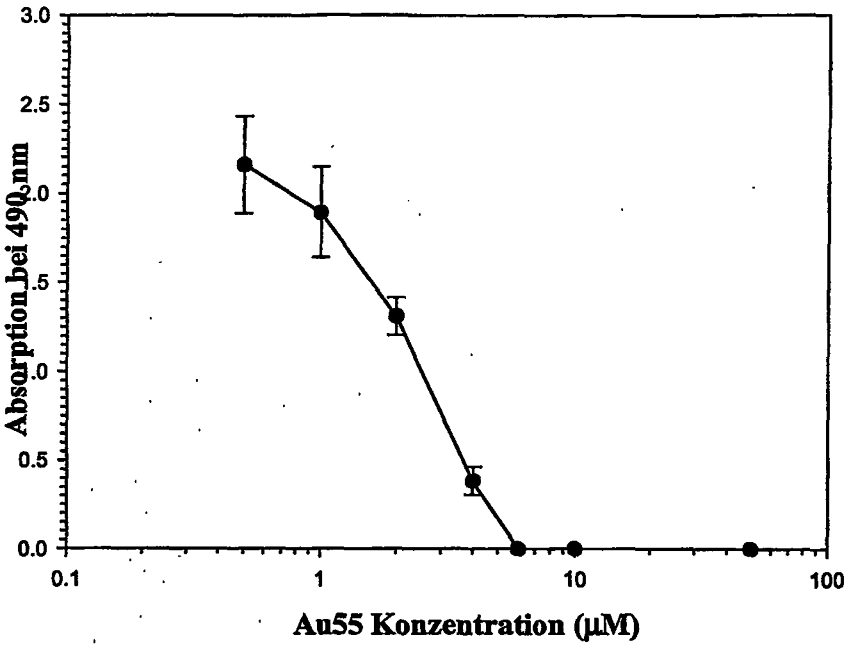
3.0
2.5
2.0
1.5
1.0
0.5
0.0
Absorption bei 490 nm
0.1
1
10
100
Au55 Konzentration (µM)

FIG. 6

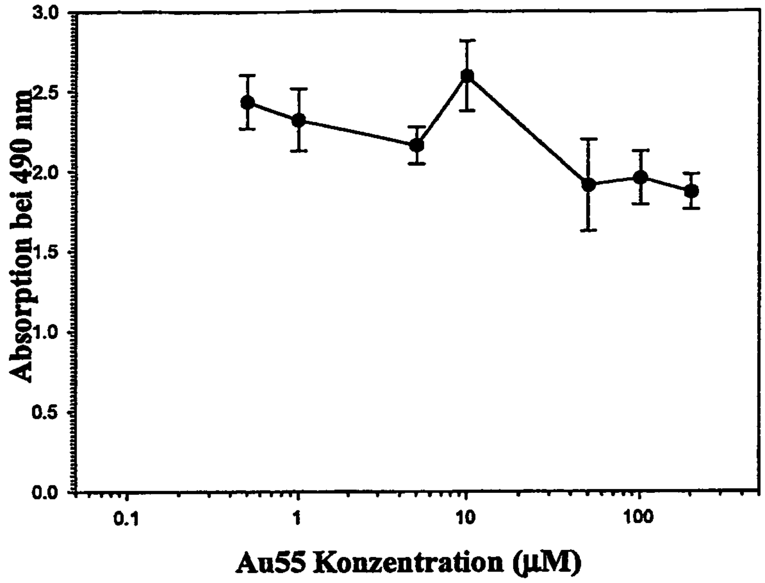
3.0
2.5
2.0
1.5
1.0
0.5
0.0
Absorption bei 490 nm
0.1
1
10
100
Au55 Konzentration (µM)

FIG. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6369206 B1
- US 5521289 B1
- US 5360819 A
- US 5521289 A
- US 5360895 A
- WO 00125841 A1
- WO 0025841 A1


### In der Beschreibung aufgeführte Nicht-Patentliteratur


- Römpp Chemielexikon. Georg Thieme Verlag, 1996, vol. 1, 773, 774
- **Willner et al.** Au-Nanoparticle Nanowires Based on DNA and Polylysine Templates. *Angew. Chem.,* 2002, vol. 114 (13), 2429-2433
- Römpp Chemielexikon. Georg Thieme Verlag, 1998, vol. 5, 4162, 4163
- **G. Schmid et al.** First Steps Towards Ordered Monolayers of Ligand-Stabilized Gold Clusters. *Angew. Chem. Int. Ed. Eng.,* 1995, vol. 34 (13,14), 1442 ff
- **G, Schmid.** Metal Clusters And Cluster Metals. *Polyhedron,* 1988, vol. 7 (22, 23), 2321-2329
- Hexachlorododecakis(triphenylphosphine)-pentapentacontagold. Inorganic Syntheses. John Wiley Verlag, 1990, vol. 27, 214-218
- Inorganic Syntheses. John Wiley Verlag, 1990, vol. 27
- Inorganic Syntheses. John Wiley Verlag, 1990, vol. 27, 214-218
- *Polyhedron,* 1988, vol. 7 (22, 23), 2321-2329
- *Angew. Chem. Int. Ed. Engl.,* 1995, vol. 34 (13, 14), 1442 ff